# EUROPÄISCHE PATENTSCHRIFT

(11) **EP 0 826 384 B1**
(45) Veröffentlichungstag und Bekanntmachung des Hinweises auf die Patenterteilung: **17.10.2001**
(21) Anmeldenummer: 97118499.9
(22) Anmeldetag: 11.03.1993
(51) Int. Cl.: A61M 1/16, A61M 1/36

(54) **Anordnung zum On-Line Spülen und Befüllen eines extrakorporalen Blutkreislaufes von Dialysemaschinen**
Arrangement for on-line cleaning and filling of an extra corporeal blood circuit of a dialysis apparatus
Dispositif de nettoyage et remplissage en ligne d'un circuit extracorporel sanguin d'une machine

(30) Priorität: 13.03.1992 DE 4208274
(43) Veröffentlichungstag der Anmeldung: 04.03.1998
(62) Teilanmeldung aus: 96103982.3
(73) Patentinhaber: MEDICAL SUPPORT GMBH, D-63110 Rodgau (DE)
(72) Erfinder: Eigendorf, Hans-Günther, Dr., 15526 Bad Saarow-Pieskow (DE)
(74) Vertreter: Flosdorff, Jürgen, Dr.

(56) Entgegenhaltungen:
- EP-A- 0 199 518
- DE-C- 3 936 785
- FR-A- 2 175 945
- US-A- 4 784 771

## Beschreibung

Die Erfindung betrifft eine Anordnung zum Spülen und Befüllen eines extrakorporalen Blutkreislaufs von Dialysemaschinen, insbesondere von Blutschlauchleitungen und Dialysatoren. Das Befüllen beinhaltet dabei die Entlüftung des extrakorporalen Blutkreislaufs, die für eine Dialysebehandlung unabdingbare Voraussetzung ist.

Als arterielle und venöse Blutschlauchleitung eines extrakorporalen Blutkreislaufs werden heutzutage üblicherweise steril verpackte Einmalartikel verwendet, die vor einer Behandlung gespült, entlüftet und befüllt werden müssen. Dies geschieht bisher mittels physiologischer Kochsalzlösungen, die mittels der maschineneigenen Blutpumpe durch das Schlauchsystem und den Dialysator gefördert werden, um den extrakorporalen Blutkreislauf von Verunreinigungen freizuspülen und unter vollständiger Entlüftung zu befüllen.

Die Verwendung der Kochsalzspülflüssigkeit hat den Nachteil, daß sie mit erheblichen Kosten verbunden ist.

Aus der FR-A-2175945 ist ein Verfahren bekannt, bei dem von einer Dialysemaschine erzeugte Dialysierflüssigkeit durch einen extrakorporalen Blutkreislauf gefördert wird. In dieser Druckschrift wird vorgeschlagen, zur Spülung des extrakorporalen Blutkreislaufs nach Beendigung einer Dialyse die beiden Schläuche des extrakorporalen Blutkreislaufs mit den Leitungen des Dialysierflüssigkeitskreislaufs zu verbinden.

Der vorliegenden Erfindung liegt die Aufgabe zugrunde, eine Anordnung anzugeben, mit der das Spülen, Entlüften und Befüllen eines extrakorporalen Blutkreislaufs von Dialysemaschinen vereinfacht ist.

Diese Aufgabe wird erfindungsgemäß durch die Merkmale des Anspruchs 1 gelöst. Vorteilhafte Weiterbildungen der Erfindung sind in den abhängigen Ansprüchen gekennzeichnet.

Gemäß der Erfindung wird die von der Dialysemaschine erzeugte bzw. aufbereitete Dialysierflüssigkeit zum Zwecke des Spülens, Entlüftens und Befüllens durch den bzw. in den extrakorporalen Blutkreislauf gefördert. Hierdurch entfällt das bisherige Erfordernis, eine in einem Beutel abgefüllte Kochsalzlösung zu diesem Zweck bereit zu stellen, der zudem von einer Bedienungsperson an die arterielle Blutschlauchleitung anzuschließen war. Es entfällt damit ferner das Erfordernis, zu dem genannten Zweck Kochsalzlösungen in größeren Mengen bereit zu halten und die geleerten Beutel zu entsorgen.

Gemäß der Erfindung wird die Dialysierflüssigkeit blutseitig des Dialysators abgenommen, wobei sie beim Durchtritt durch die Membran filtiert wird. Hiermit ist die Keimfreiheit der Spül- und Befüllflüssigkeit zuverlässig gewährleistet.

Gemäß der Erfindung wird die dem Dialysator entnommene Dialysierflüssigkeit von der maschineneigenen Blutpumpe durch den extrakorporalen Blutkreislauf gepumpt, was nachfolgend in näheren Einzelheiten erläutert wird. Bei dieser Ausgestaltung der Erfindung wird die Saugseite der arteriellen Blutschlauchleitung vorzugsweise mittels eines Einmalartikels an dem Dialysator konektiert, vorzugsweise an der Stelle, an der bei der Dialysebehandlung die venöse Blutschlauchleitung angeschlossen wird. Das Blutpumpensegment der arteriellen Blutschlauchleitung wird in die maschineneigene Blutpumpe, vorzugsweise eine Rollerpumpe, eingelegt, und das andere, freie Ende der arteriellen Blutschlauchleitung wird vorzugsweise über einen weiteren Einmalartikel mit dem Einlauf der venösen Blutschlauchleitung konnektiert. Die venöse Blutschlauchleitung durchläuft auf übliche Weise einen Luftdetektor. Das andere Ende der venösen Blutschlauchleitung wird stromabwärts des Dialysators an den Dialsysierflüssigkeitskreislauf angeschlossen, vorzugsweise mittels eines weiteren Einmalartikels an ein Probeentnahmeventil dieses Dialysierflüssigkeitskreislaufs.

Wenn die Blutpumpe in Betrieb gesetzt wird, wird Dialysierflüssigkeit über die semipermeable Membran des Dialysators in die Blutschlauchleitungen gezogen. Dabei gelangt nur sterile und keimfreie Spülflüssigkeit in den extrakorporalen Blutkreislauf. Die Spülflüssigkeit und die in dem extrakorporalen Kreislauf zuvor befindliche Luft werden stromabwärts des Dialysators in den Dialysierflüssigkeitskreislauf der Dialysemaschine eingeführt, womit bilanz- und sonstige maschinenseitigen Störungen vermieden sind.

Nach einem zusätzlichen Vorschlag der Erfindung ist vorgesehen, daß ein Sterilfilter stromaufwärts des Dialysators in dem Dialysierflüssigkeitskreislauf angeordnet wird, um die Sicherheit in Bezug auf die erforderliche Keimfreiheit zusätzlich zu erhöhen.

Nach einem weiteren Gesichtspunkt der Erfindung wird vorgeschlagen, daß die als Spülflüssigkeit verwendete Dialysierflüssigkeit nach Beendigung einer Dialysebehandlung zur Freispülung des extrakorporalen Blutkreislaufes verwendet wird, wozu es zweckmäßig ist, diese Flüssigkeit beim Spülvorgang in einen Beutel abzuführen. Das Freispülen ist notwendig, um nach der Dialysebehandlung das Blut vollständig zum Patienten zurückzuführen, was bisher ebenfalls mit Hilfe einer Kochsalzlösung geschieht.

Ein weiterer erfindungsgemäßer Vorschlag geht dahin, die extrakorporalen Komponenten so zu reinigen, daß sie wiederverwendbar sind. Hierzu kann die Anordnung so getroffen werden, wie sie weiter oben beschrieben ist. Bei einem dialysemaschinenspezifischen Reinigungsprogramm wird durch die Förderung der Blutpumpe Reinigungsflüssigkeit über die semipermeable Membran des Dialysators in den extrakorporalen Kreislauf gezogen, wodurch dieser entsprechend gereinigt wird.

Selbstverständlich liegt es im Rahmen der Erfindung, die extrakorporalen Komponenten weiterhin als Einmalartikel einzusetzen.

Weitere Merkmale, Vorteile und Einzelheiten der Erfindung ergeben sich aus der nachfolgenden Beschreibung einiger bevorzugter Ausführungsformen sowie anhand der Zeichnung. Dabei zeigen auf rein schematische Weise:
- Fig. 1: eine herkömmliche Anordnung;
- Fig. 2: eine Ausführungsform der erfindungsgemäßen Anordnung.

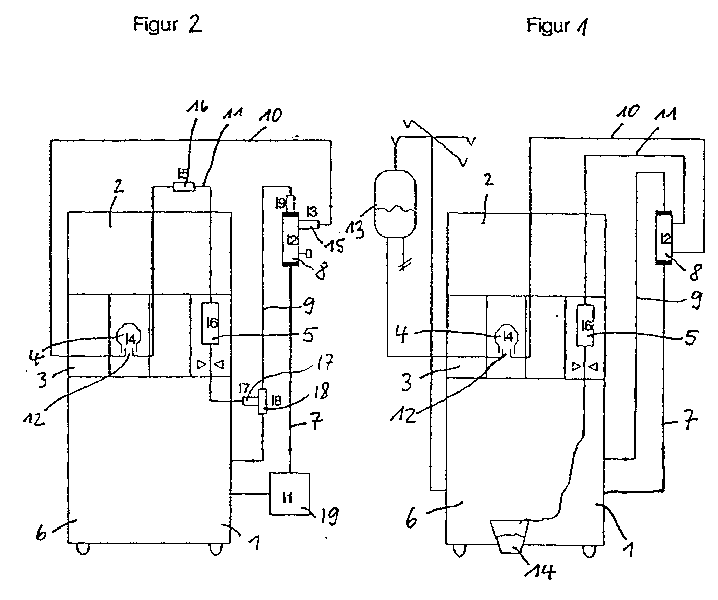

Es wird zunächst auf Fig. 1 Bezug genommen, um das bisher übliche Verfahren zum Spülen, Entlüften und Befüllen eines extrakorporalen Blutkreislaufs zu erläutern. Die Fig. zeigt eine eigentliche Dialysemaschine 1 mit der oberen Überwachungsebene 2, der mittleren Ebene 3, die eine Blutpumpe 4 und einen Luftdetektor 5 enthält, und mit einer unteren Ebene 6, die den Hydraulikteil aufweist.

Die Dialysemaschine 1 ist über eine Zuleitung 7 mit dem Dialysator 8 verbunden, von dem eine Rückleitung 9 zur Dialysemaschine 1 zurückführt, um einen Dialysierflüssigkeitskreislauf zu schließen.

Zum Zwecke einer Dialysebehandlung werden eine arterielle Blutschlauchleitung 10 und eine venöse Blutschlauchleitung 11 an den dargestellten Stellen an dem Dialysator 8 konnektiert. Ein Blutpumpensegment 12 der arteriellen Blutschlauchleitung 10 wird in die Blutpumpe 4 eingelegt, während die venöse Blutschlauchleitung 11 über den Luftdetektor 5 führt.

Zur Vorbereitung einer Dialysebehandlung müssen die Blutschlauchleitungen 10 und 11 freigespült werden und der extrakorporale Blutkreislauf muß entlüftet und befüllt werden. Hierzu ist es bisher üblich, das freie Ende der arteriellen Blutschlauchleitung 10 mit dem Ausgang eines eine Kochsalzlösung enthaltenden Beutels 13 zu verbinden. Die Blutpumpe 4 fördert die Kochsalzlösung durch die arterielle Blutschlauchleitung 10, den Dialysator 8, die venöse Blutschlauchleitung 11 und den Luftdetektor 5, um schließlich die Spülflüssigkeit in einen offenen Behälter 14 abzuführen.

Es wird nun auf Fig. 2 Bezug genommen, in der die Bauteile, die mit denjenigen gemäß Fig. 1 übereinstimmen, mit denselben Bezugszeichen gekennzeichnet sind. Auf ihre Wiederholung wird aus Gründen der Übersichtlichkeit verzichtet.

Bei dieser Ausführungsform der Erfindung wird die arterielle Blutschlauchleitung 10 mittels eines Einmalartikels 15 an der Stelle an dem Dialysator 8 konnektiert, an der gemäß Fig. 1 die venöse Blutschlauchleitung 11 angeschlossen ist. Das Blutpumpensegment 12 der arteriellen Blutschlauchleitung 10 ist mittels eines Einmalartikels 16 mit der venösen Blutschlauchleitung 11 verbunden. Die venöse Blutschlauchleitung 11 führt wiederum über den Luftdetektor 5 und ist mittels eines Einmalartikels 17 an ein Probeentnahmeventil 18 angeschlossen.

In der Leitung 7 des Dialysierflüssigkeitskreislaufs ist ein Sterilfilter 19 angeordnet, der die Sicherheit der Keimfreiheit der Spülflüssigkeit weiter erhöht.

Durch Förderung der Blutpumpe 4 gelangt Dialysierflüssigkeit aus dem Dialysierflüssigkeitskreislauf 7, 8, 9 in die arterielle Schlauchleitung 10 und die venöse Schlauchleitung 11, von wo sie in den Dialysierflüsigkeitskreislauf (bei 18) zurückgegeben wird, um bilanz- und sonstige maschinenseitigen Störungen zu vermeiden.

## Patentansprüche

1. Anordnung zum On-Line Spülen und Befüllen eines extrakorporalen Blutkreislaufs von Dialysemaschinen (1), insbesondere von Blutschlauchleitungen (10, 11) und Dialysatoren (8),
**dadurch gekennzeichnet,**
**daß** die arterielle Blutschlauchleitung (10) blutseitig an dem Dialysator (8) konnektiert ist, daß das Blutpumpensegment (12) der arteriellen Blutschlauchleitung in die Blutpumpe (4) eingelegt ist, daß das andere Ende der arteriellen Blutschlauchleitung (10) mit dem Einlauf der venösen Blutschlauchleitung (11) verbunden ist und daß das andere Ende der venösen Blutschlauchleitung (11) stromabwärts des Dialysators (8) in den Dialysierflüssigkeitskreislauf einmündet.

2. Anordnung nach Anspruch 1,
**dadurch gekennzeichnet, daß** die Saugseite der arteriellen Blutschlauchleitung an der Stelle an dem Dialysator (8) konnektiert ist, an der bei der Dialysebehandlung die venöse Blutschlauchleitung (11) angebracht ist.

3. Anordnung nach Anspruch 1 oder 2,
**dadurch gekennzeichnet, daß** als Anschlüsse für die Leitungen (10, 11) Einmalartikel (15,16,17) verwendet werden.

4. Anordnung nach einem der Ansprüche 1 bis 3,
**dadurch gekennzeichnet, daß** das andere Ende der venösen Leitung (11) an eine Probeentnahmeventil (18) in dem Dialysierflüssigkeitskreislauf konnektiert ist.

5. Anordnung nach einem der Ansprüche 1 bis 4,
**dadurch gekennzeichnet, daß** die venöse Blutschlauchleitung (11) über einen Luftdetektor (5) führt.

6. Anordnung nach einem der Ansprüche 1 bis 5,
**dadurch gekennzeichnet, daß** ein Sterilfilter (19) in der Zuleitung des Dialysierflüssigkeitskreislaufs stromaufwärts des Dialysators (8) angeordnet ist.

## Claims

1. Arrangement for the on-line flushing and filling of an extracorporeal blood circuit of dialysis machines (1), in particular of blood tube lines (10, 11) and dialysers (8),
**characterised in**
**that** the arterial blood tube line (10) is connected on the blood side to the dialyser (8), that the blood pump segment (12) of the arterial blood tube line is placed in the blood pump (4), that the other end of the arterial blood tube line (10) is connected to the inlet of the venous blood tube line (11), and that the other end of the venous blood tube line (11) leads into the dialysing fluid circuit downstream of the dialyser (8).

2. Arrangement according to Claim 1,
**characterised in that** the intake side of the arterial blood tube line is connected to the dialyser (8) at the point at which the venous blood tube line (11) is fitted for dialysis treatment.

3. Arrangement according to Claim 1 or 2,
**characterised in that** disposable articles (15, 16, 17) are used as connections for the lines (10, 11).

4. Arrangement according to any one of Claims 1 to 3,
**characterised in that** the other end of the venous line (11) is connected to a sampling valve (18) in the dialysing fluid circuit.

5. Arrangement according to any one of Claims 1 to 4,
**characterised in that** the venous blood tube line (11) passes via an air detector (5).

6. Arrangement according to any one of Claims 1 to 5,
**characterised in that** a sterile filter (19) is arranged in the feed line of the dialysing fluid circuit upstream of the dialyser (8).

## Revendications

1. Agencement de nettoyage et de remplissage en ligne d'un circuit extracorporel sanguin de machines de dialyse (1), notamment de tuyaux souples de sang (10, 11) et de dialyseurs (8), **caractérisé en ce que** le tuyau de sang artériel (10) est connecté, côté sang, au dialyseur (8), **en ce que** le segment de pompe de sang (12) du tuyau de sang artériel est placé dans la pompe de sang (4), **en ce que** l'autre extrémité du tuyau de sang artériel (10) est reliée à l'entrée du tuyau de sang veineux (11) et **en ce que** l'autre extrémité du tuyau de sang veineux (11) débouche en aval du dialyseur (8) dans le circuit de liquide de dialyse.

2. Agencement selon la revendication 1, **caractérisé en ce que** le côté aspiration du tuyau de sang artériel est connecté au dialyseur (8) à l'emplacement où, lors du traitement par dialyse, est disposé le tuyau de sang veineux (11).

3. Agencement selon la revendication 1 ou 2, **caractérisé en ce que** des articles à usage unique (15, 16, 17) sont utilisés comme raccords pour les tuyaux (10, 11).

4. Agencement selon l'une des revendications 1 à 3, **caractérisé en ce que** l'autre extrémité du tuyau veineux (11) est connectée à une vanne de prélèvement d'échantillon (18) dans le circuit de liquide de dialyse.

5. Agencement selon l'une des revendications 1 à 4, **caractérisé en ce que** le tuyau de sang veineux (11) passe sur un détecteur d'air (5).

6. Agencement selon l'une des revendications 1 à 5, **caractérisé en ce qu'**un filtre stérile (19) est disposé dans le conduit d'amenée du circuit de liquide de dialyse en amont du dialyseur (8).
